**Europäisches Patentamt**

**European Patent Office**

**Office européen des brevets**

(11) Veröffentlichungsnummer: **0 136 530**
**B1**

(19)

(12) **EUROPÄISCHE PATENTSCHRIFT**

(45) Veröffentlichungstag der Patentschrift:
**01.06.88**

(51) Int. Cl.⁴: **A 61 N 1/40**, A 61 N 5/02,
A 61 F 7/00

(21) Anmeldenummer: **84110179.3**

(22) Anmeldetag: **27.08.84**

(54) **Bestrahlungsvorrichtung zur Behandlung von lebendem Gewebe mit elektromagnetischen Wellen.**

(30) Priorität: **12.09.83 DE 3332843**

(43) Veröffentlichungstag der Anmeldung:
**10.04.85 Patentblatt 85/15**

(45) Bekanntmachung des Hinweises auf die Patenterteilung:
**01.06.88 Patentblatt 88/22**

(84) Benannte Vertragsstaaten:
**AT BE CH DE FR GB IT LI LU NL SE**

(56) Entgegenhaltungen:
**EP - A - 0 011 019**
**DE - A - 2 721 757**
**DE - A - 2 827 003**
**DE - A - 2 850 248**
**US - A - 3 800 802**
**US - A - 3 952 751**
**US - A - 4 197 851**
**US - A - 4 230 129**
**US - A - 4 403 618**

(73) Patentinhaber: **Broers, Dieter-Ernst, Soorstrasse 10,**
**D-1000 Berlin 19 (DE)**

(72) Erfinder: **Broers, Dieter-Ernst, Soorstrasse 10,**
**D-1000 Berlin 19 (DE)**

(74) Vertreter: **Neubauer, Hans–Jürgen, Dipl.-Phys.,**
**Fauststrasse 30, D-8070 Ingolstadt (DE)**

## Beschreibung

Die Erfindung betrifft ein Gerät zur Behandlung von lebendem Gewebe und Organen mit elektromagnetischen Wellen zur therapeutischen Beeinflussung bei Erkrankungen bakterieller, viröser und nervlicher Art sowie bei Tumorbildung gemäss dem Oberbegriff des Anspruchs 1.

Bestrahlungsvorrichtungen, die mit elektromagnetischen Wellen arbeiten, sind bereits bekannt.

Für eine Zerstörung von Tumorgewebe werden seit langem ionisierende elektromagnetische Strahlen angewendet. Eine solche Behandlung hat den Nachteil, dass nicht nur das erkrankte Gewebe bestrahlt wird, sondern auch gesundes Gewebe, durch das die Strahlung hindurchgeht, geschädigt wird, da die hier verwendete Strahlung relativ hart und energiereich ist.

Bei einer anderen bekannten Behandlungsmethode von Tumoren (DE-B 2 634 628, DE-B 2 304 500, DE-B 2 306 922) wird das gegenüber dem gesunden Gewebe schon wärmere Tumorgewebe so überhitzt, dass dieses zerstört wird. Auch bei einer solchen Behandlung wird gesundes Gewebe geschädigt, insbesondere treten schmerzhafte Verbrennungen der Hautpartien auf, durch die die elektromagnetischen Wellen zugeführt werden.

Es ist bekannt (DE-B 2 748 780), durch eine Änderung der elektrischen oder elektrochemischen Umgebung einer lebenden Zelle oder eines Gewebes einen therapeutischen Effekt zu erzielen. Beispielsweise kann das Knochenwachstum nach Knochenbrüchen dadurch vorteilhaft beeinflusst werden. Hierzu ist angegeben, dass zwei verschiedene, elektrische Code-Signale anzuwenden sind, die das Wachstum stimulieren. In der genannten Form sind diese aber ungeeignet, das Wachstum etwa vom Tumorgewebe zu unterdrücken oder gar derartiges Gewebe selektiv abzutöten.

Aus der US-A 3 789 832 ist ein Methode zur Krebsfrüherkennung durch Empfang von Mikrowellen charakteristischer Frequenzen bekannt, die von den Krebszellen ausgesandte Strahlung darstellt. Aufbauend auf diese Erkenntnis wurde bereits vorgeschlagen (DE-B 2 423 399), Tumorgewebe selektiv mittels elektromagnetischer Strahlung dadurch zu beeinflussen, dass die von der Krebszelle ausgesandte Strahlung empfangen und durch Abstrahlung dem Tumor wieder zugeführt wird. Hier soll angeblich das Wachstum der Tumorzellen durch Resonanz beeinflussbar sein, während gesunde Zellen, bei denen keine Resonanz stattfindet, nicht beeinflusst werden sollen. In der genannten Schrift sind jedoch weder Angaben über Frequenzen oder Leistungen noch über klinische Ergebnisse gemacht. Neuere Erkenntnisse deuten eher darauf hin, dass durch eine verstärkte Strahlung des Tumorgewebes die Eigenfrequenz gesunder Zellen gewaltsam auf die Frequenz der Tumorzellen verschoben wird, so dass die bisher noch gesunden Zellen selbst zu unkontrolliertem Wachstum gelangen, wodurch das Gegenteil dessen erreicht würde, was in der DE-OS 2 423 399 versprochen wird.

Eine weitere Bestrahlungseinrichtung, die mit elektromagnetischen Strahlen arbeitet, ist aus der EP-A1 0 011 019 bekannt, die zur Beeinflussung der Zellenaktivität dient. Bei dieser Einrichtung (Fig. 1) wird ein Hochfrequenzgenerator eingesetzt, der mit einer Frequenz von 27,12 MHz schwingt. Dem Hochfrequenzoszillator nachgeschaltet ist ein Verstärker, an den ein Taktgenerator (Modulationsgenerator) angeschlossen ist. Der Taktgenerator erzeugt im wesentlichen Rechteckimpulse, die dazu dienen, den Ausgang des Verstärkers periodisch zu unterbrechen. Dadurch werden dem nachgeschalteten Leistungsverstärker bzw. einer angeschlossenen Antenne zeitlich unterbrochene Wellenzugstücke zugeführt. Als charakteristische Werte für die Taktfrequenz werden dort 50 bis 1000 Hz genannt, wobei die Impulsdauer bzw. Dauer eines Wellenzugs ca. 10 bis 100 Mikrosekunden lang sein soll. Da feststeht, dass es elektromagnetische Wellen in Zellen gibt und andererseits durch Bestrahlung mit elektromagnetischen Wellen die Zellen auch beeinflussbar sind, wird der bekannten Einrichtung eine Zellbeeinflussung nicht abgesprochen. Aufgrund intensiver Versuche und theoretischer Überlegungen hat sich allerdings gezeigt, dass eine Bestrahlungseinrichtung für eine effektive Therapie für andere Frequenzbereiche ausgelegt sein muss.

Ausgehend vom letztgenannten Stand der Technik wird daher die Aufgabe der Erfindung darin gesehen, eine gattungsgemässe Bestrahlungseinrichtung so weiterzubilden, dass damit ein grosser therapeutischer Effekt erzielbar ist.

Diese Aufgabe wird mit den Merkmalen des Anspruchs 1 gelöst.

Gemäss Anspruch 1 ist in der Bestrahlungsvorrichtung eine Hochfrequenzoszillatorstufe vorgesehen, die in einem einstellbaren Frequenzbereich von 100 bis 200 MHz arbeitet. Diese Frequenz wird mit einer von 1 Hz bis 1000 Hz einstellbaren Niederfrequenz moduliert und einem Taktgenerator zugeführt, der mit einer Taktfrequenz von 0,5 Hz bis 40 Hz einstellbar arbeitet. Die am Ausgang des Taktgenerators anliegenden unterbrochenen Wellenzugteile werden einem Endverstärker zugeführt, an den eine Sendeantenne angeschlossen ist. Im Abstrahlbereich der Sendeantenne wird das zu behandelnde Gewebe angebracht oder liegt der Patient. Eine Änderung der Abstrahlrichtung ergibt dabei zusätzlich eine Polarisationsänderung. Die Antenne kann auch in das zu bestrahlende Gewebe implantiert sein.

In Versuchen wurde festgestellt, dass es elektromagnetische Wellen in Zellen gibt. Diese Strahlung war, von wenigen Ausnahmen abgesehen, z.B. bei einigen Algen, Bakterien, Protozoen, in allen untersuchten pflanzlichen und tierischen Geweben nachzuweisen, auch dem menschlichen Gewebe. Die Intensität liegt stets in der ebenfalls im Versuch ermittelten Grössenordnung von einigen 100 bis 1000 Photonen pro Sekunde und pro cm² Zellkulturfläche. Die Energien der Photonen erstreckten sich auf den Wellenbereich von 800 bis etwa 350 Nanometer mit einem Maximum bei ca. 550 Nanometer.

Es wird davon ausgegangen, dass diese Strahlung auch zur Kommunikation zwischen den Zellen dient und beispielsweise auch das Wachstum reguliert. Ein Hinweis darauf ist die starke Änderung in der Intensität und im Spektrum der Strahlung von Krebsgewebe. Krebszellen sind somit als resonanzgestörte Zellen anzusehen, deren chronische Resonanzstörung zu dauerhaftem Wachstum führt. Das verstärkte Wachstum führt zu einer noch stärkeren Resonanzstörung, so dass eine Selbstgeneration nicht mehr möglich ist. Es ist auch ohne weiteres denkbar, dass eine gesunde Zelle durch eine Strahlung von Mikroben aus dem Schwingungsgleichgewicht gebracht wird und damit krank wird.

Beobachtungen haben gezeigt, dass der Krebs in der Mehrzahl der Fälle bei Personen mittleren Alters, etwa von 50 Jahren an, und bei Greisen auftritt. Daraus kann geschlossen werden, dass er sich in «abgenutztem» Gewebe ausbildet. Es ist daher anzunehmen, dass die Anfälligkeit für Krebs in einer Veränderung des Blutes oder der Zellen besteht, die sich mit jenen Altersstufen einstellt, bzw. durch die Veränderung der zellulären Schwingungen, die durch die Veränderung der Kapazität der Zellen bewirkt wird.

Ausgehend von den vorstehenden Betrachtungen wurde die erfindungsgemässe Bestrahlungsvorrichtung entwickelt und in verschiedenen Kliniken in einer Reihe von Versuchen getestet. Insbesondere hat sich gezeigt, dass eine Bestrahlung mit der hochfrequenten Grundfrequenz von 100 MHz bis 200 MHz, die niederfrequenz-moduliert ist und anschliessend zerhackt wird, ein hervorragender therapeutischer Effekt erzielt wird. Die Wellenlänge der hochfrequenten Grundfrequenz entspricht damit beispielsweise etwa der gestreckten Helix eines DNA-Moleküls, die mit ca. 2 m festgestellt wurde. Dies könnte ebenfalls eine Bekräftigung der vorstehenden Überlegungen zur Resonanzstörung bei erkrankten Zellen sein.

Mit der erfindungsgemässen Bestrahlungsvorrichtung wird somit eine Strahlungsquelle zur Verfügung gestellt, die eine elektromagnetische Schwingung geeigneter Frequenz und Amplitude an das kranke Gewebe heranbringt, die Zelle in ihren ursprünglichen Normalzustand überführt und dadurch die Gesundheit fördert und wiederbringt.

Die erfindungsgemässe Bestrahlungsvorrichtung ruft biologische Reaktionen in den Zellen hervor und erzeugt insbesondere modulierte, elektromagnetische Wellen, die mit den Grundbestandteilen einer gesunden Zelle in Resonanz stehen. Dadurch wird ein magnetisches Wechselfeld im Körper des Patienten erzeugt, dessen Wirbelströme günstige Ladungsverschiebungen in den Zellmembranen zur Folge haben und im vegetativen Nervensystem günstige Reizungen hervorrufen, die vorhandene Blockierungen abbauen. Alle diese Einflüsse laufen insgesamt auf eine Stärkung des Immunsystems hinaus.

Ein besonderer Vorteil neben der guten therapeutischen Wirkung besteht bei der erfindungsgemässen Bestrahlungsvorrichtung darin, dass nur elektromagnetische Wellen mit einer sehr geringen Leistung im Milliwatt- bis ca. 10-Watt-Bereich erzeugt werden, die die angeführten biologischen Wirkungen bereits hervorrufen. Eine Strahlungsschädigung des Patienten, insbesondere von gesundem Gewebe, ist daher ausgeschlossen. Vielmehr wird nach der Anwendung in wenigen Stunden eine Besserung im Allgemeinbefinden des Patienten festgestellt, die vom Patienten auch subjektiv als gesteigertes Wohlbefinden empfunden wird.

Zu der hervorragenden therapeutischen Wirkung wird ein Beispiel angeführt: Nach erfolgreich abgeschlossenen Versuchsreihen des Erfinders an Pflanzen (Geranien) und Tieren (Mäusen) fand die Bestrahlungseinrichtung ihren ersten praktischen Einsatz am Menschen in einer Klinik in Aachen. Ein 72jähriger männlicher Patient litt seit über 20 Jahren an chronischem Asthma und bekam dreimal täglich einen Asthmaanfall. Er konnte kaum einen Satz ohne Atemnot sprechen. Für die Behandlung wurde eine Antenne unter die Bettdecke gelegt und die Bestrahlungsdauer auf täglich 4 × 20 Minuten angesetzt. Nach lediglich 5 Behandlungstagen konnte der Patient bereits wieder laufen und sogar Treppen steigen, ohne in Atemnot zu geraten; vom zweiten Behandlungstag ab wurde überhaupt kein Asthmaanfall mehr festgestellt. Entsprechende Versuche sind zwischenzeitlich mit einer Reihe von Patienten erfolgreich durchgeführt worden. Eine mobile Bestrahlungseinrichtung kann zur Nachbehandlung direkt am Körper getragen werden, wobei die Antenne entweder als Halsband oder als Hüftgürtel (jeweils isoliert) am Körper getragen wird.

Gemäss Anspruch 2 soll bei der Taktung das Verhältnis zwischen Wellenzugdauer und der Pausenzeit 1:1 oder kleiner sein, was zu guten Erfolgen führt.

Mit den Merkmalen des Anspruchs 3 wird einerseits eine Anpassung der Sendeantenne an die Sendefrequenz erreicht und andererseits die Möglichkeit geschaffen, unterschiedlich lange Antennen (Halsband, Hüftgürtel usw.) einzusetzen.

In Anspruch 4 ist der bevorzugte Frequenzbereich angegeben, bei dem eine noch gesteigerte Wirkung erkannt wurde, so dass gegebenenfalls die Bestrahlungseinrichtung nur auf diesen Frequenzbereich ausgelegt sein könnte.

Mit den Merkmalen des Anspruchs 5 wird eine vorteilhafte Ausgestaltung und Weiterbildung des Erfindungsgegenstandes angegeben, wodurch eine erhebliche Steigerung der therapeutischen Wirkung zu erreichen ist. Dazu wird eine weitere Niederfrequenzoszillatorstufe vorgesehen, die von 1 Hz bis 1000 Hz einstellbar ist und die mit einer Spule als Abstrahlelement verbunden ist. Die Spule und zugleich die Sendeantenne sind bei einer Behandlung auf das zu behandelnde Gewebe oder einen Patienten zu richten. Dabei ist, wie in Anspruch 6 angeführt, die magnetische Vorzugsrichtung der Spule zu bevorzugen.

Es hat sich gezeigt, dass insbesondere im modulierten Hochfrequenzbereich in der Regel eine grössere Effizienz bei negativen Wellenteilen erzielt wird. Nach den durchgeführten Versuchen sprechen nur ca. 10% der Patienten besser auf positive Wellenteile an. Dies ist allerdings noch abhängig vom Indikationsbereich. Es ist daher gemäss Anspruch 7 eine umschaltbare Einrichtung zur Wahl von positiven oder negativen Amplitudenwerten in der Bestrahlungsvorrichtung enthalten.

Gute Ergebnisse wurden mit der in Anspruch 8 beanspruchten «Wellenschaukel» erzielt, wobei die erzeugte und über die Spule abgestrahlte Niederfrequenz kontinuierlich zwischen Werten von 7 Hz bis 12 Hz pendelt. Diese Frequenz liegt im Bereich des α-Rhythmus. Es hat sich gezeigt, dass die grösste Effektivität bei einer Übereinstimmung dieser Niederfrequenzabstrahlung mit der Frequenz des α-Rhythmus erreicht wird. Da der α-Rhythmus aufwendig zu messen ist (EEG) und in der Frequenz variiert, wird mit der Wellenschaukel erreicht, dass zumindest zeitweise eine Übereinstimmung mit dem jeweiligen α-Rhythmus erfolgt.

Es hat sich gezeigt, dass die Wirkung mit den in Anspruch 9 beanspruchten Wellenformen mit hohem Oberwellenanteil verbessert werden kann.

Die Sendeleistung der Bestrahlungseinrichtung kann innerhalb der in Anspruch 10 genannten sehr kleinen Werte liegen, bei denen eine Gewebeschädigung ausgeschlossen ist. Diese geringen Bestrahlungsleistungen (20 mW bis 180 mW) sind wesentlich, da sich gezeigt hat, dass die Effektivität bei höheren Leistungen wieder abnimmt.

Mit Anspruch 11 ist eine weitere zweckmässige Ausgestaltung der Erfindung für die Automatisierung der Behandlung genannt.

Anhand zweier Ausführungsbeispiele wird die Erfindung mit weiteren Merkmalen, Einzelheiten und Vorteilen näher erläutert.

Es zeigen

Fig. 1 das Schaltbild einer Ausführungsform der Erfindung,

Fig. 2 das Blockschaltbild einer weiteren Ausgestaltung und Weiterbildung,

Fig. 3 bis 5 Schaltpläne gemäss dem Blockschaltbild aus Fig. 2.

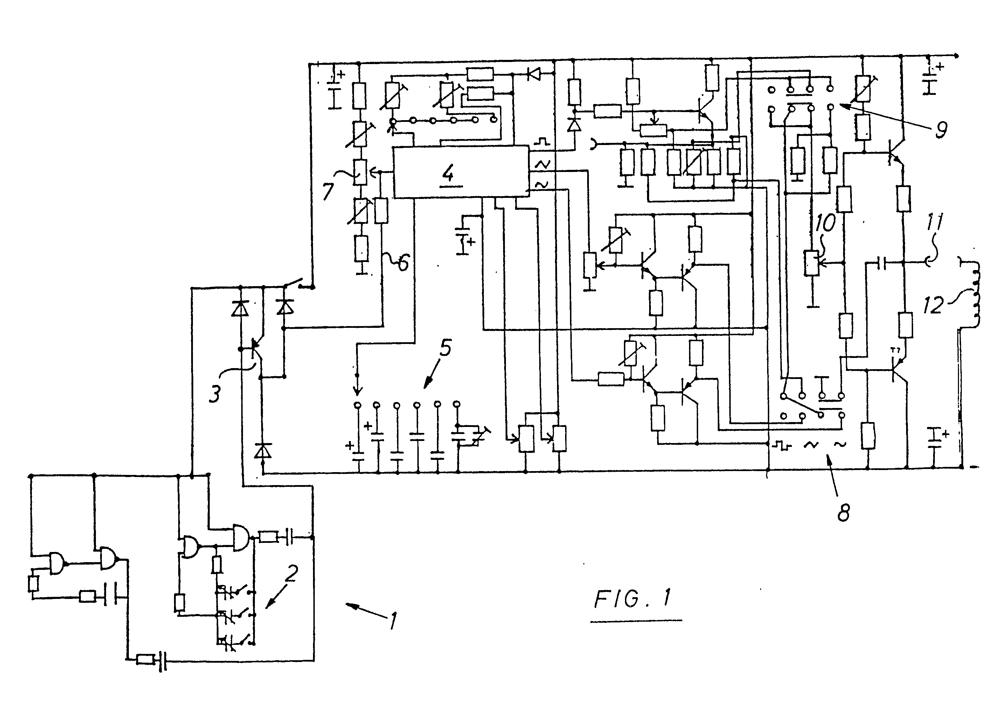

In Fig. 1 ist der Schaltplan einer Bestrahlungseinrichtung dargestellt, bei der ein Niederfrequenzoszillator 1 durch Flip-Flop-Stufen gebildet ist. Die Einschaltdauer und damit die Frequenz des NF-Oszillators 1 ist durch drei zuschaltbare Kondensatoren 2 einstellbar. Am Ausgang dieses Schaltungsteils liegt ein Transistor 3 als Anpassungs- und Verstärkungsglied.

Eine integrierte Schaltung 4 erzeugt die Hochspannungsfrequenz, die mit den schaltbaren Kapazitäten 5 im vorgegebenen Bereich einstellbar ist. Für eine Modulation der Hochfrequenzschwingung ist die Niederfrequenzschwingung über die Leitung 6 der integrierten Schaltung 4 zugeführt und über den Widerstand 7 in der Leistung einstellbar. Mit dem Schalter 8 ist die Kurvenform wählbar zwischen Sinus, Rechteck oder Sägezahn. Mit dem Schalter 9 ist eine Grundeinstellung der Ausgangsleistung möglich (TTL; x 1; x 0,1). Mit dem Potentiometer 10 wird eine Feineinstellung der Ausgangsleistung durchgeführt. Am Ausgang 11 ist eine stilisiert angedeutete Sendeantenne 12 angeschlossen, die bevorzugt als Rahmenantenne ausgebildet ist. Die nicht weiter bezeichneten Bauteile dienen der Verstärkung, Anpassung und Taktung des Signals.

Die gezeigte Schaltung bzw. die damit realisierte Bestrahlungsvorrichtung hat folgende Funktion: Mit der integrierten Schaltung 4 wird ein Hochfrequenzsignal im Bereich von 100 bis 200 MHz einstellbar erzeugt. Dieses ist mit dem Niederfrequenzsignal aus dem Niederfrequenzoszillator 1 moduliert. Dieses modulierte Signal wird mit der über den Schalter 8 wählbaren Kurvenform getaktet der Sendeantenne 12 zugeführt, in deren Abstrahlbereich der Patient oder das zu behandelnde Gewebe liegt.

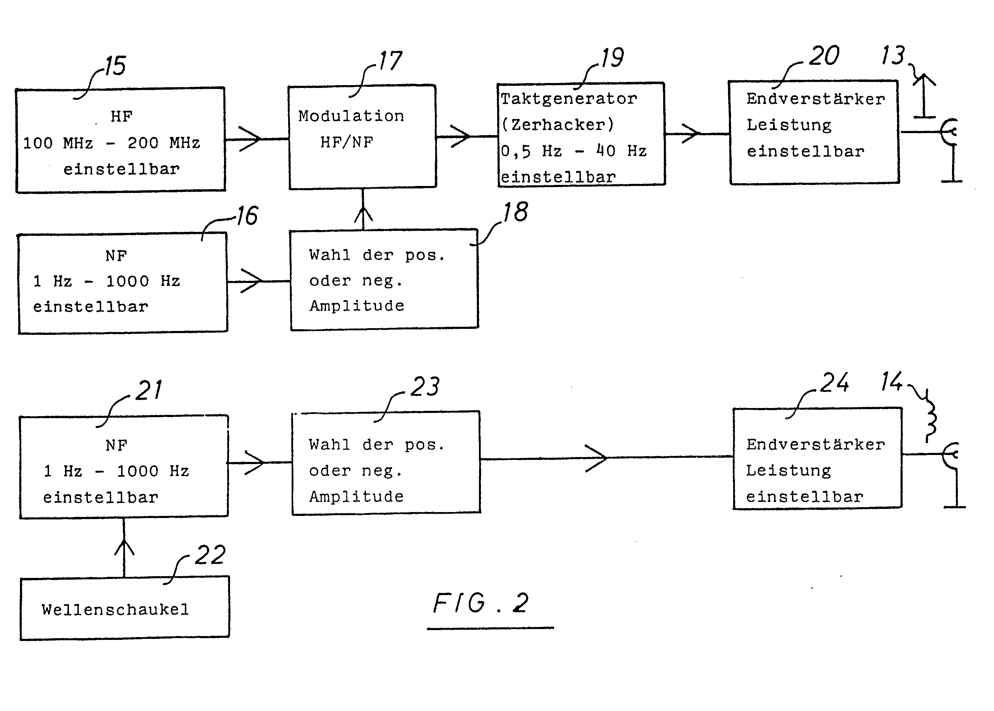

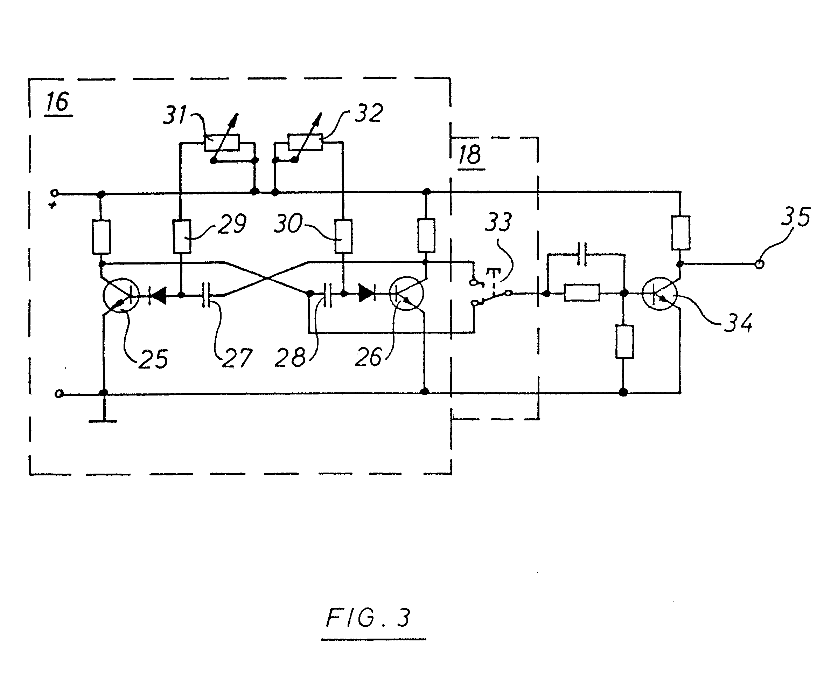

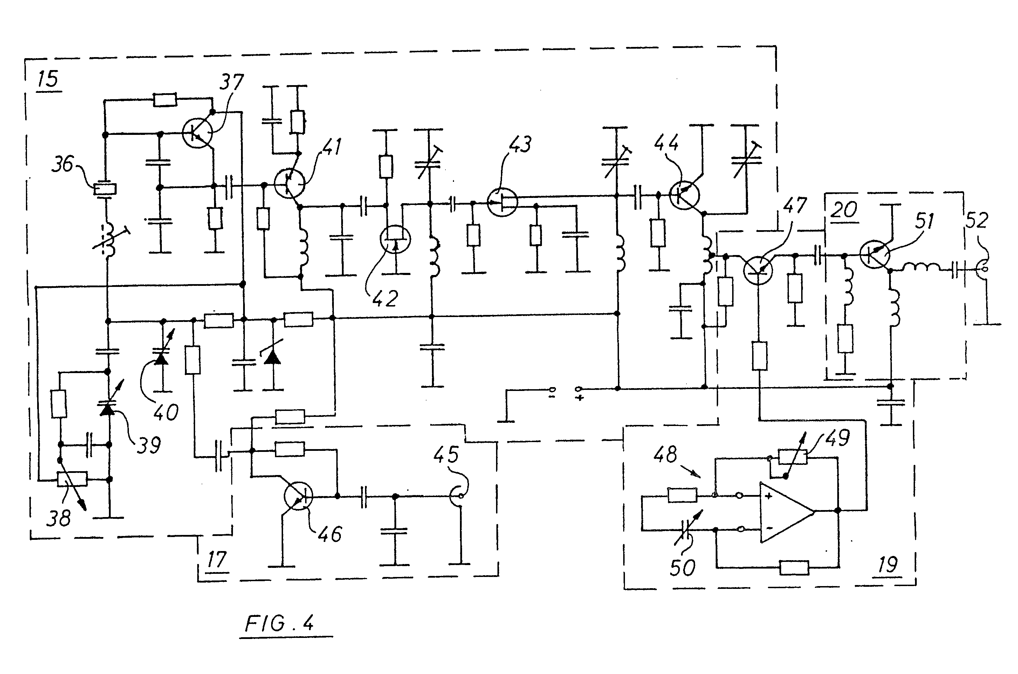

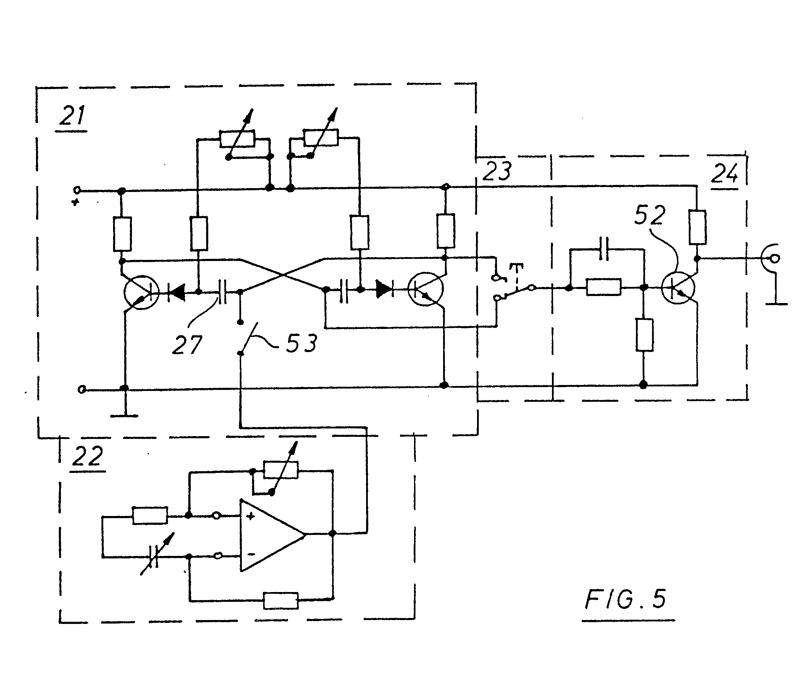

In Fig. 2 ist ein Blockschaltbild dargestellt, bei dem der obere mit der Sendeantenne 13 verbundene Teil im wesentlichen der Schaltung nach Fig. 1 entspricht. In den Fig. 3 und 4 ist ein anderer Schaltungsaufbau dieses Teiles wiedergegeben. Der untere mit einer Spule 14 verbundene Teil betrifft eine weitere Ausgestaltung, die im Detail im Schaltplan nach Fig. 5 gezeigt ist.

Ein Hochfrequenzoszillator 15 (100 MHz bis 200 MHz) und ein Niederfrequenzoszillator 16 (1 Hz bis 1000 Hz) liegen parallel und ihre Ausgänge sind in einer Modulationseinheit 17 zusammengeführt, wobei in einer Einheit 18 eine Wahl der positiven oder negativen Amplitude erfolgen kann. Das modulierte Signal wird von einem Taktgenerator 19 (0,5 Hz bis 40 Hz) zerhackt und einem Endverstärker 20 zugeführt, an den die Sendeantenne 13 angeschlossen ist.

In einer zweiten Linie, deren Teile gegebenenfalls auch in einem separaten Gehäuse untergebracht sein könnten, ist ein Niederfrequenzoszillator 21 (1 Hz bis 1000 Hz) angeordnet, der durch die mit Wellenschaukel 22 bezeichnete Einheit zu sich periodisch ändernden Schwingungen zwischen 7 bis 12 Hz erregbar ist. Am Ausgang des Niederfrequenzoszillators 21 ist wieder eine Einheit 23 zur Wahl der positiven oder negativen Amplitude angebracht, deren Ausgang mit einem weiteren Endverstärker 24 verbunden ist, an den die Spule 14 angeschlossen ist.

Es werden nun die Schaltungsteile im einzelnen beschrieben, wobei die Bezugszeichen des Blockschaltbildes verwendet werden:

In Fig. 3 ist der Niederfrequenzoszillator 16 und die Einheit 18 dargestellt. Zwei Transistoren 65, 66 bilden den Generator. Die frequenzbestimmenden Bauteile sind die Kondensatoren 27, 28 in Verbindung mit den Widerständen 29, 30 und den Potentiometern 31, 32. Mit den Potentiometern 31 und 32 ist sowohl die Frequenz als auch das Taktverhältnis zwischen Impuls- und Pausenzeit einstellbar. Der Oszillator liefert bei gleicher Einstellung an den Potentiometern 31, 32 etwa Rechtecksignale, wird diese Einstellung gegeneinander verdreht, verändert sich die Impulsform in Richtung auf Nadelimpulse. Auch die Kondensatoren 27, 28 könnten durchstimmbar gewählt werden, wobei sich bei ungleicher Einstellung ebenfalls das Tastverhältnis ändert.

Mit dem Bezugszeichen 18 ist die Einheit zur Wahl der positiven oder negativen Amplitude dargestellt, die im wesentlichen aus dem Schalter 33 besteht.

Ein nachgeschalteter Transistor 34 verbessert die Rechteckform des Signales und entlastet den Generator, so dass die eingestellte Frequenz des Generators 16 stabilisiert wird. Am Punkt 35 steht dann das Niederfrequenzsignal zur Verfügung.

In Fig. 4 ist die oberste Linie des Blockschaltbildes gemäss Fig. 2 ausgeführt. Der Hochfrequenzoszillator 15 wird durch einen Quarz 36 und einen Transistor 37 gebildet und ist mit dem Potentiometer 38 abstimmbar. Mit diesem Potentiometer 38 ist die Abstimmspannung für Kapazitätsdioden 39, 40 einstellbar und eine Frequenzeinstellung im Bereich von ca. 18 bis 20 MHz möglich. Dem Oszillator 36, 37 ist eine Pufferstufe nachgeschaltet, die im wesentlichen aus dem Transistor 41 besteht und zur Anpassung und Entlastung des Oszillators dient. Nach der Pufferstufe folgen drei Frequenzverdoppelungseinheiten, wobei der Transistor 42 (FET) eine erste Frequenzverdoppelung, der Transistor 43 (FET) eine zweite Frequenzverdoppelung und der Transistor 44 eine dritte Frequenzverdoppelung durchführt.

Das Hochfrequenzsignal ist bereits moduliert, da mit dem Eingang 45 der Ausgang 35 aus Fig. 3 verbunden ist. Der Transistor 46 der Modulationseinheit 17 bewirkt lediglich eine Verstärkung. Das modulierte Hochfrequenzsignal liegt damit an einem Transistor 47 an, der zusammen mit einem Generator 48 den Taktgenerator 19 bzw. Zerhacker 19 bildet, wobei über den Transistor 47 das Ausgangssignal zum Endverstärker 20 unterbrochen wird. Mit dem Potentiometer 49 und dem Kondensator 50 ist die Frequenz und das Tastverhältnis des Taktgenerators 19 einstellbar.

Ein Transistor 51 bildet die Endstufe 20 des Hochfrequenzsenders, an dessen Ausgang 52 eine Sendeantenne angeschlossen ist.

Der bisher beschriebene Hochfrequenzteil hat folgende Funktion: Mit dem Potentiometer 38 wird nach Einschalten des Geräts die gewünschte Hochfrequenz und mit den Potentiometern 31, 32 die gewünschte Niederfrequenz einschliesslich des Taktverhältnisses eingestellt. Mit dem Schalter 33 wird je nach Indikation und Patient die positive oder negative Amplitude gewählt. Weiter wird mit dem Potentiometer 49 und dem Kondensator 50 die gewünschte Frequenz und das Taktverhältnis des Taktgenerators eingestellt, wodurch das Gerät für eine Behandlung im modulierten Hochfrequenzbereich eingestellt ist.

Es folgt nun die Beschreibung der Niederfrequenzlinie (Fig. 2, unterer Teil) anhand der Fig. 5.

Der Niederfrequenzoszillator 21 und die Einheit zur Wahl der positiven oder negativen Amplitude 23 sind mit den in Fig. 3 dargestellten Einheiten 16 und 18 im wesentlichen gleich aufgebaut, so dass hierauf Bezug genommen wird. Der Einheit 23 ist die Endstufe 24 nachgeschaltet, die im wesentlichen aus einem Transistor 52 besteht, an den eine (nicht dargestellte) Spule 14 anschliessbar ist.

Zur Realisierung einer Wellenschaukel, d.h. zu einem Pendeln des Niederfrequenzoszillators 21 zwischen 7 und 12 Hz ist die Einheit 22 vorgesehen, die im grundsätzlichen Aufbau dem Generator 48 aus Fig. 4 entspricht, so dass hierauf Bezug genommen wird. Dieser Generator ist über einen Schalter 53 zuschaltbar und verändert somit die Spannung am Kondensator 27, wodurch die vorgenannte Frequenzpendelung bewirkt wird. Mit der Einstellung der in Fig. 5 enthaltenen Potentiometer und Schalter ist auch der Niederfrequenzteil für eine Behandlung vorbereitet.

**Patentansprüche**

1. Bestrahlungsvorrichtung zur Behandlung von lebendem Gewebe mit elektromagnetischen Wellen, mit einer Hochfrequenzoszillatorstufe (15) zur Erzeugung eines Hochfrequenzwellenzuges, mit einem nachgeschalteten Taktgenerator (19) als Zerhacker, der den Hochfrequenzwellenzug periodisch unterbricht und mit einem Endverstärker (20), an den eine Sendeantenne (13) anschliessbar ist, in derem Abstrahlbereich das zu behandelnde Gewebe bzw. ein Patient postierbar ist, dadurch gekennzeichnet, dass die Hochfrequenzoszillatorstufe (15) eine im Bereich von 100 bis 200 MHz einstellbare Frequenz erzeugt, dass eine Niederfrequenzoszillatorstufe (16) vorgesehen ist, die eine im Bereich von 1 Hz bis 1000 Hz einstellbare Frequenz erzeugt, dass der Ausgang der Hochfrequenzoszillatorstufe (15) und der Ausgang der Niederfrequenzoszillatorstufe (16) in einer Modulationseinheit (17) zusammengeführt sind, so dass ein modulierter Wellenzug entsteht, und dass der modulierte Wellenzug dem Taktgenerator (19) zugeführt wird und der Taktgenerator (19) in einer Taktfrequenz von 0,5 Hz bis 40 Hz einstellbar ist.

2. Bestrahlungsvorrichtung nach Anspruch 1, dadurch gekennzeichnet, dass das Verhältnis zwischen der Dauer des am Taktgenerator (19) durchgelassenen Wellenzuges zu der Pausenzeit 1:1 oder kleiner ist.

3. Bestrahlungsvorrichtung nach Anspruch 1 oder 2, dadurch gekennzeichnet, dass die Länge der Sendeantenne (12; 13) der eingestellten Hochfrequenz bzw. deren Wellenlänge $\lambda$ oder ganzen Teilen wie

$$\lambda, \frac{\lambda}{2} \frac{\lambda}{4}$$

etc. entspricht.

4. Bestrahlungsvorrichtung nach Anspruch 1, dadurch gekennzeichnet, dass die Hochfrequenzoszillatorstufe (15) eine Frequenz von 140 bis 160 MHz erzeugt.

5. Bestrahlungsvorrichtung nach einem der Ansprüche 1 bis 4, dadurch gekennzeichnet, dass eine zweite Niederfrequenzoszillatorstufe (21) enthalten ist, die eine im Bereich von 1 Hz bis 1000 Hz einstellbare Frequenz erzeugt, dass der zweiten Niederfrequenzoszillatorstufe (21) ein zweiter Endverstärker (24) nachgeschaltet ist, an den eine Spule (14) als Abstrahlelement anschliessbar ist, wobei das zu behandelnde Gewebe bzw. ein Patient gleichzeitig in den Abstrahlbereich der Sendeantenne (13) und in den Abstrahlbereich der Spule (14) bringbar ist.

6. Bestrahlungsvorrichtung nach Anspruch 5, dadurch gekennzeichnet, dass das zu behandelnde Gewebe bzw. der Patient in der magnetischen Vorzugsrichtung der Spule (14) liegt.

7. Bestrahlungsvorrichtung nach einem der Ansprüche 1 bis 6, dadurch gekennzeichnet, dass wenigstens eine umschaltbare Einrichtung (18, 23) für positive oder negative Amplitudenwerte enthalten ist.

8. Bestrahlungsvorrichtung nach einem der Ansprüche 5 bis 7, dadurch gekennzeichnet, dass die zweite Niederfrequenzoszillatorstufe (21) als Wellenschaukel (22) ausgebildet ist, d.h. die erzeugte Niederfrequenz kontinuierlich zwischen Werten von 7 Hz bis 12 Hz pendelt.

9. Bestrahlungsvorrichtung nach einem der Ansprüche 1 bis 8, dadurch gekennzeichnet, dass die Oszillatoren (15, 16, 21; 1, 4) Impulse bzw. Wellenzüge mit hohem Oberwellenanteil erzeugen, insbesondere Rechteck-, Sägezahn- und Nadelimpulse.

10. Bestrahlungsvorrichtung nach einem der Ansprüche 1 bis 9, dadurch gekennzeichnet, dass die Sendeleistung an dem Endverstärker (20, 24) von 20 mW bis 180 mW, bevorzugt 50 mW, einstellbar ist.

11. Bestrahlungsvorrichtung nach einem der Ansprüche 1 bis 10, dadurch gekennzeichnet, dass ein Zeitschaltwerk vorgesehen ist, das für die zeitliche Behandlungsdauer und/oder die Verstellung von erzeugten Frequenzen oder Sendeleistungen programmierbar ist.

**Claims**

1. A radiation device for the treatment of living tissue with electromagnetic waves, with a high frequency oscillator stage (15) for the generation of a high frequency wave train, with a clock generator (19) down-line as a chopper which periodically interrupts the high frequency wave train and with an output amplifier (20) whereto there may be connected a transmitting aerial (13) into whose radiation range there may be positioned the tissue to be treated, or a patient, characterised in that the high frequency oscillator stage (15) produces a frequency which may be set in the range of 100 to 200 MHz, that provision is made for a low frequency oscillator stage (16) which produces a frequency that may be set within the range of 1 Hz to 1000 Hz that the output of the high frequency oscillator stage (15) and the output of the low frequency oscillator stage (16) are brought together in a modulator unit (17) so that a modulated wave train is generated and that the modulated wave train is passed to the clock generator (19) and that the clock generator (19) may be set to a pulse frequency of 0.5 Hz to 40 Hz.

2. A radiation device according to Claim 1, characterised in that the ratio of the period of the wave train allowed to pass at the clock generator (19), and of the break interval is 1:1, or less.

3. A radiation device according to Claims 1 or 2, characterised in that the length of the transmitting aerial (12; 13) corresponds to the high frequency set, or to its wavelength $\lambda$, or to whole parts, such as $\lambda/2$, $\lambda/4$ etc.

4. A radiation device according to Claim 1, characterised in that the high frequency oscillator stage (15) produces a frequency of 140 to 160 MHz.

5. A radiation device according to one of Claims 1 to 4, characterised in that a second low frequency oscillator stage (21) is contained therein which produces a frequency that may be set within the range of 1 Hz to 1000 Hz, that the second low frequency oscillator stage (21) is followed down-line by a second output amplifier (24) to which a coil (24) may be connected as a radiation element, the tissue to be treated, or a patient, being capable of being simultaneously brought into the radiation range of the transmitting aerial (13) and into the radiation range of the coil (14).

6. A radiation device according to Claim 5, characterised in that the tissue to be treated, or a patient, lies in the preferred magnetic direction of the coil (14).

7. A radiation device according to one of Claims 1 to 6, characterised in that at least one switchable device (18, 23) for positive or negative amplitude values is contained therein.

8. A radiation device according to one of Claims 5 to 7, characterised in that the second low frequency oscillator stage (21) is designed as a wave swing, that is to say that the lower frequency produced continuously oscillates between values from 7 Hz to 12 Hz.

9. A radiation device according to one of Claims 1 to 8, characterised in that the oscillators (15, 16, 21, 1, 4) produce pulses or wave trains with a high harmonic content, in particular rectangular, sawtooth and needle pulses.

10. A radiation device according to one of Claims 1 to 9, characterised in that the transmission power at the output amplifier (20, 24) is adjustable from 20 mW to 180 mW, preferably to 50 mW.

11. A radiation device according to one of Claims 1 to 10, characterised in that provision is made for a time switch which can be programmed for the control of the duration of the treatment period and/or for the variation of the frequencies or transmission powers.

## Revendications

1. Dispositif d'irradiation pour le traitement de tissus vivants par des ondes électromagnétiques, comportant un étage oscillateur à haute fréquence (15) pour la production d'un train d'ondes à haute fréquence, un générateur de rythme (19) monté en aval, servant de hacheur qui interrompt périodiquement le train d'ondes à haute fréquence, et un amplificateur terminal (20) auquel peut être connectée une antenne émettrice (13) dans la zone de rayonnement de laquelle peut être placé le tissu à traiter ou un patient, caractérisé en ce que l'étage oscillateur à haute fréquence (15) produit une fréquence qui est réglable dans la gamme de 100 à 200 MHz, en ce qu'il est prévu un étage oscillateur à basse fréquence (16) qui produit une fréquence réglable dans la gamme de 1 Hz à 1000 Hz, en ce que la sortie de l'étage oscillateur à haute fréquence (15) et la sortie de l'étage oscillateur à basse fréquence (16) sont reliées à une unité de modulation (17), de telle sorte qu'il soit formé un train d'ondes modulé, en ce que le train d'ondes modulé est transmis au générateur de rythme (19) et en ce que le générateur de rythme (19) est réglable à une fréquence entre 0,5 Hz et 40 Hz.

2. Dispositif d'irradiation selon la revendication 1, caractérisé en ce que le rapport entre la durée du train d'ondes transmis au générateur de rythme (19) et le temps de pause est égal ou inférieur à 1:1.

3. Dispositif d'irradiation selon la revendication 1 ou 2, caractérisé en ce que la longueur de l'antenne émettrice (12, 13) correspond à la haute fréquence réglée ou à la longueur d'one $\lambda$ ou à des fractions entières telles que $\lambda/2$, $\lambda/4$ etc. de celle-ci.

4. Dispositif d'irradiation selon la revendication 1, caractérisé en ce que l'étage oscillateur à haute fréquence (15) produit une fréquence de 140 à 160 MHz.

5. Dispositif d'irradiation selon l'une quelconque des revendications 1 à 4, caractérisé en ce qu'il contient un second étage oscillateur à basse fréquence (21) qui produit une fréquence réglable dans la gamme de 1 Hz à 1000 Hz, et en ce qu'il est monté, à la suite du second oscillateur à basse fréquence (21), un second amplificateur terminal (24) auquel peut être connectée une bobine (14) servant d'élément de rayonnement, le tissu à traiter ou un patient pouvant être placé en même temps dans la zone de rayonnement de l'antenne émettrice (13) et dans la zone de rayonnement de la bobine (14).

6. Dispositif d'irradiation selon la revendication 5, caractérisé en ce que le tissu à traiter ou le patient est placé dans la direction magnétique préférentielle de la bobine (14).

7. Dispositif d'irradiation selon l'une quelconque des revendications 1 à 6, caractérisé en ce qu'il contient au moins un dispositif réversible (18, 23) pour des valeurs d'amplitude positives ou négatives.

8. Dispositif d'irradiation selon l'une quelconque des revendications 5 à 7, caractérisé en ce que le second étage oscillateur à basse fréquence (21) est réalisé sous forme de bascule (22), c'est-à-dire que la basse fréquence produite oscille en permanence entre des valeurs de 7 Hz à 12 Hz.

9. Dispositif d'irradiation selon l'une quelconque des revendications 1 à 8, caractérisé en ce que les oscillateurs (15, 16, 21; 1, 4) produisent des impulsions ou des trains d'impulsions à forte proportions d'ondes harmoniques, en particulier des impulsions carrées, en dents de scie et en pointe.

10. Dispositif d'irradiation selon l'une quelconque des revendications 1 à 9, caractérisé en ce que la puissance d'émission au niveau de l'amplificateur terminal (20, 24) est réglable de 20 mW à 180 mW, de préférence à 50 mW.

11. Dispositif d'irradiation selon l'une quelconque des revendications 1 à 10, caractérisé en ce qu'il est prévu un interrupteur à minuterie qui est programmable pour la durée temporelle du traitement et/ou pour le réglage des fréquences produites ou des puissances d'émission.

FIG. 1

| 15 | | 17 | | 19 | | 20 | 13 |
|---|---|---|---|---|---|---|---|
| HF<br>100 MHz - 200 MHz<br>einstellbar | → | Modulation<br>HF/NF | → | Taktgenerator<br>(Zerhacker)<br>0,5 Hz - 40 Hz<br>einstellbar | → | Endverstärker<br>Leistung<br>einstellbar | |

| 16 | | 18 |
|---|---|---|
| NF<br>1 Hz - 1000 Hz<br>einstellbar | → | Wahl der pos.<br>oder neg.<br>Amplitude |

| 21 | | 23 | | | 24 | 14 |
|---|---|---|---|---|---|---|
| NF<br>1 Hz - 1000 Hz<br>einstellbar | → | Wahl der pos.<br>oder neg.<br>Amplitude | → | | Endverstärker<br>Leistung<br>einstellbar | |

| 22 |
|---|
| Wellenschaukel |

_FIG.2_

0 136 530

FIG.3

FIG.4

F I G. 5